(19) Europäisches Patentamt
European Patent Office
Office européen des brevets

(11) Veröffentlichungsnummer: **0 226 536**
**A2**

(12) # EUROPÄISCHE PATENTANMELDUNG

(21) Anmeldenummer: **86810545.3**

(22) Anmeldetag: **26.11.86**

(51) Int. Cl.[4]: **C 07 D 251/70**
A 01 N 47/18

(30) Priorität: **02.12.85 CH 5130/85**

(43) Veröffentlichungstag der Anmeldung:
**24.06.87 Patentblatt 87/26**

(84) Benannte Vertragsstaaten:
**AT BE CH DE ES FR GR IT LI LU NL SE**

(71) Anmelder: **CIBA-GEIGY AG**
**Klybeckstrasse 141**
**CH-4002 Basel (CH)**

(72) Erfinder: **Gehret, Jean-Claude, Dr.**
**Im Aeschfeld 12**
**CH-4147 Aesch (CH)**

**Kristiansen, Odd, Dr.**
**Delligrabenstrasse 7**
**CH-4313 Möhlin (CH)**

(54) **(Di)alkoxycarbonyl-amino-s-triazin-Derivate gegen tier- und pflanzenparasitäre Schädlinge.**

(57) Es werden (Di)alkoxycarbonyl-amino-s-triazine und ihre schwefelanalogen Vertreter der Formel I

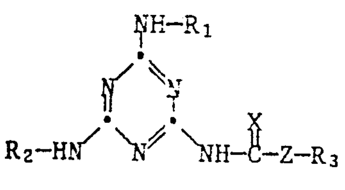

(I)

worin
$R_1$ $C_1$-$C_6$-Alkyl oder $C_3$-$C_6$- Cycloalkyl bedeutet;
$R_2$ für Wasserstoff, $C_1$-$C_6$-Alkyl, $C_3$-$C_6$-Cycloalkyl oder die Gruppe -C(X)-Z-$R_3$ steht;
$R_3$ für $C_1$-$C_6$-Alkyl, $C_1$-$C_6$-Haloalkyl, $C_2$-$C_4$-Alkenyl oder $C_2$-$C_4$-Haloalkenyl steht;
X für Sauerstoff oder Schwefel steht; und
Z für Sauerstoff oder Schwefel steht;
unter Einschluss deren Säureadditionssalze beschrieben. Ferner ihre Herstellung aus den zugrundeliegenden Diamino-s-triazine sowie ihre Verwendung gegen tier- und pflanzenpathogene Schädlinge.

EP 0 226 536 A2

**Beschreibung**

(Di)alkoxycarbonyl-amino-s-triazin-Derivate gegen tier- und pflanzenparasitäre Schädlinge

Die vorliegende Erfindung betrifft (Di)alkoxycarbonyl-amino-s-triazin-Derivate und ihre schwefelanalogen Vertreter der nachstehenden Formel I, ihre Herstellung und ihre Verwendung gegen tier-oder pflanzenpathogene Parasiten und Insekten, ferner Schädlingsbekämpfungsmittel, die mindestens eine dieser Verbindungen als Wirkstoff enthalten.

Die erfindungsgemässen Verbindungen haben die Formel I

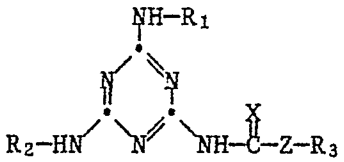

(I)

worin

$R_1$ $C_1$-$C_6$-Alkyl oder $C_3$-$C_6$-Cycloalkyl bedeutet;
$R_2$ für Wasserstoff, $C_1$-$C_6$-Alkyl, $C_3$-$C_6$-Cycloalkyl oder die Gruppe -C(X)-Z-$R_3$ steht;
$R_3$ für $C_1$-$C_6$-Alkyl, $C_1$-$C_6$-Haloalkyl, $C_2$-$C_4$-Alkenyl oder $C_2$-$C_4$-Haloalkenyl steht;
X für Sauerstoff oder Schwefel steht; und
Z für Sauerstoff oder Schwefel steht;
unter Einschluss deren Säureadditionssalze.

Unter dem Begriff Alkyl selbst oder als Bestandteil eines anderen Substituenten sind im folgenden je nach Zahl der angegebenen Kohlenwasserstoffatome beispielsweise folgende geradkettigen Gruppen zu verstehen: Methyl, Ethyl, Propyl, Butyl, Pentyl und Hexyl, sowie ihre verzweigten Isomeren, wie z.B. Isopropyl, Isobutyl, tert.-Butyl, sek.-Butyl, Isopentyl, usw. Alkenyl steht z.B. für Vinyl, Propenyl-(1), Allyl, Butenyl-(1), Butenyl-(2), Butenyl-(3), usw. Die Vorsilbe Halo besagt, dass der entsprechende Substituent ein- oder mehrfach durch ein oder mehrere, gleiche oder verschiedene Halogenatome substituiert ist. Beispiele für Haloalkyl sind: $CCl_3$, $CF_3$, $CBr_3$, $CHCl_2$ $CHF_2$, $CHBr_2$, $CH_2Cl$, $CH_2F$, $CH_2Br$, CHClF, CFClBr, $C_2F_5$, $C_2Cl_5$, $CH_2CF_3$, $C(CH_3)_2CF_3$, usw. Beispiele für Haloalkenyl sind: $CCl_2 = CCl_2$, $CH_2 = CCl_2$, $CF_2 = CF_2$, $CH_2 = CF_2$, $CHCl = CCl_2$, $CHCl = CClF$, $CH_2$-$CH_2 = CCl_2$ usw. Cycloalkyl steht z.B. für Cyclopropyl, Cyclobutyl, Cyclopentyl oder Cyclohexyl.

Unter dem Begriff Säureadditionssalze von Verbindungen der Formel I sind Additionssalz anorganischer und organischer Säuren zu verstehen, durch Anlagerung einer equivalenten Menge einer salzbildenden Säure an das Basismolekül entstehen.

Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie z.B. Essigsäure, Triflfuoressigsäure, Trichloressigsäure, Propionsäure, Glykolsäure, Thiocyansäure, Milchsäure, Bernsteinsäure, Zitronensäure, Ameisensäure, Benzolsulfonsäure, p-Toluolsulfonsäure, Methansulfonsäure, Salicylsäure, p-Aminosalicylsäure, Phthalsäure, 2-Phenoxybenzoesäure oder 2-Acetoxybenzoesäure.

Die Verbindungen der Formel I liegen bei Raumtemperatur überwiegend als stabile Feststoffe vor, die einen Schmelzpunkt von ca. 50° bis ca. 220° C aufweisen. Sie besitzen sehr wertvolle parasitizide Eigenschaften und lassen sich zur kurative und präventiven Bekämpfung eine Reihe von Pflanzen- und Tierparasiten und Insekten einsetzen, so insbesondere gegen Dipterenlarven. Gegenüber anderen Triamino-s-triazin-derivaten zeigen sie den Vorteil, dass sie bei Applikation an ein Nutztier im wesentlichen im Kot ausgeschieden werden und dort durch Entfaltung einer intensiven larviziden Wirkung der Verbreitung des Parasiten verhindern. Bei herkömmlichen Triamino-s-triazinen geht ein Grossteil der Aktivsubstanz durch Urinausscheidung verloren.

Auf Grund ihrer ausgeprägten Wirkung sind folgende Gruppen von Verbindungen der Formel I bevorzugt.

a) Verbindungen der Formel I, worin
$R_1$ $C_3$-Alkyl oder Cylcopropyl bedeutet;
$R_2$ für Wasserstoff, $C_1$-$C_3$-Alkyl, Cyclopropyl oder C(O)O$R_3$ steht;
$R_3$ $C_1$-$C_4$-Alkyl, $C_1$-$C_4$-Chloroalkyl, $C_2$-$C_4$-Alkenyl oder $C_2$-$C_4$-Haloalkenyl bedeutet; und
X und Z für Sauerstoff stehen.

b) Verbindungen der Formel I, worin
$R_1$ Isopropyl oder Cyclopropyl bedeutet;
$R_2$ für Wasserstoff oder $C_1$-$C_3$-Alkyl steht;
$R_3$ Methyl, Ethyl, n-Propyl, n-Butyl oder $C_2$-$C_4$-Alkenyl bedeutet; und
X und Z für Sauerstoff stehen.

Bevorzugte Einzelsubstanzen sind z.B.
2-Cyclopropylamino-4-amino-6-allyloxycarbonylamino-s-triazin und seine Säureadditionssalze,
2-Isopropylamino-4-amino-6-allyloxycarbonylamino-s-triazin, seine Säureadditionssalze,

2-Cyclopropylamino-4-amino-6-methoxycarbonxylamino-s-triazin und seine Säureadditionssalze,
2-Cyclopropylamino-4-amino-6-ethoxycarbonylamino-s-triazin und seine Säureadditionssalze,
2-Cyclopropylamino-4-amino-6-n-butoxycarbonylamino-s-triazin und seine Säureadditionssalze,
wobei die Chlorwasserstoffsäureadditionssalze besonders hervorzuheben sind.

Die Verbindungen der Formel I werden hergestellt indem man eine Verbindung der Formel II

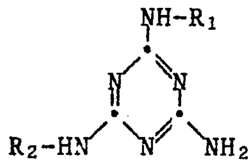

(II),

vorzugsweise in Gegenwart einer Base, mit einer ausreichenden Menge eines reaktionsfähigen Säurederivates der Säure III

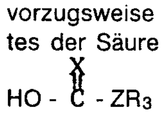

(III)

bei Temperaturen von 0° bis 50°C, vorzugsweise 15° bis 30°C, versetzt und das resultierende Reaktionsgemisch ca. 5 bis 12 Stunden bei 10° bis 100°C hält, wobei in den Formeln II und III die Substituenten $R_1$, X, Z und $R_3$ die unter Formel I angegebenen Bedeutungen haben und $R_2$ für Wasserstoff, $C_1$-$C_6$-Alkyl oder $C_3$-$C_6$-Cycloalkyl steht.

Reaktionsfähige Säurederivate der Säure III sind z.B. ihr Anhydrid oder Säurehalogenid, insbesondere ihr Säurechlorid oder -bromid. Als ausreichend wird die Menge an reaktionsfähigem Säurederivate angesehen, die zu dem gewünschten Produkt führt. D.h. in den Fällen, in denen $R_2$ für Wasserstoff, $C_1$-$C_6$-Alkyl oder $C_3$-$C_6$-Cycloalkyl steht und somit nur an einer der $NH_2$-Gruppen substituiert werden soll, setzt man 1 Aequivalen oder einen geringen Ueberschuss des Säurederivates von III ein, während wenn $R_2$ für -C(X)-$ZR_3$ steht und somit beide $NH_2$-Gruppen substituiert werden sollen, empfiehlt sich der Einsatz von 2 Aequivalenten oder eines leichten Ueberschusses des Säurederivates von III. Im allgemeinen geht man so vor, dass man das Derivat von III (ca. 1,1 eq. für $R_2 = H$, $C_1$-$C_6$-Alkyl oder $C_3$-$C_6$-Cycloalkyl und ca. 2,2 eq. für $R_2$ = -C(X)-$ZR_3$) in wenig Lösungsmittel löst und zu einem Gemisch von II, einer Base und einem inerten Lösungsmittel oder Lösungsmittelgemisch langsam bei 0° bis 50°C, vorzugsweise 15° bis 30° zutropfen lässt und mehrere Stunden, z.B. über Nacht bei 10 bis 100°C, bei $R_2 = H$, $C_1$-$C_6$-Alkyl oder $C_3$-$C_6$-Cycloalkyl vorzugsweise bei 15° bis 30°C, bei $R_2$ = C(X)-$ZR_3$ vorzugsweise bei 40° bis 80°C zu Ende reagieren lässt. Nach beendeter Reaktion lässt man das Gemisch Raumtemperatur errreichen, filtriert von festen Niederschlägen ab und entfernt das Lösungsmittel z.B. im Vakuum. Der Rückstand wird im einem üblichen organischen Lösungsmittel aufgenommen, mit Wasser und gesättigter Kochsalzlösung gewaschen, über einem geeigneten Trockenmittel (z.B. Natriumsulfat oder Magnesiumsulfat) getrocknet, filtriert und eingeengt. Nach dem Entfernen des Lösungsmittels kann das Produkt durch übliche Methoden gereinigt werden, so z.B. durch Auflösen in einem Lösungsmittel und anschliessendem Ausfällen mit einem anderen Lösungsmittel. Es können auch chromatographische Reinigungsmethoden eingesetzt werden.

Für die Umsetzung von II mit III eignen sich ein Prinzip alle reaktionsinerten Lösungsmittel, die man üblicherweise bei Acylierungsreaktionen einsetzt. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisoproopylether, tert.-Butylmethylether usw.) Anisol, Dioxan, Tetrahydrofuran; Nitrile wie Acetonitril, Pripionitril; N,N-dialkylierte Amide wie Dimethylformamid; Dimethylsulfoxid; Ketone wie Aceton, Diethylketon, Methylethylketon und Gemische solcher Lösungsmittel untereinander.

Als geeignete Basen kommen organische und anorganische Basen in Betracht, z.B. teriäre Amine wie Trialkylamine (Trimethylamin, Triethylamin, Tripropylamin, Diisopropylethylamin usw.), Pyridin und Pyridinbasen (4-Dimethylaminopyridin, 4-Pyrrolidylaminopyridin usw.), N-Methylpyrrolidon etc. sowie Oxide und Hydroxide, Carbonate und Hydrogencarbonate von Alkali- und Erdalkalimetallen sowie Alkaliacetate.

Die Ausgangsverbindungen der Formeln II sowie die Säuren III und deren reaktionsfähige Derivate sind allgemein bekannt oder lassen sich analog zu den bekannten Vertretern herstellen.

Es wurde nun überraschenerweise festgestellt, dass die Verbindungen der Formel I eine ausgeprägte Dipterenlarven, ausüben. Die Verbindungen der Formel I wirken vor allem auf die juvenilen Stadien der Insekten. Die Wirkung besteht in einem Absterben der Eilarven oder in der Verhinderung des Schlüpfens von Adulten aus den Puppen. Die Wirkung der Verbindungen der Formel I ist nicht mit der Wirkungsweise von klassichen Insektiziden, Chemosterilantien oder Juvenilhormonanalogen zu vergleichen.

Die Wirkstoffe der Formel 1 werden zur Bekämpfung von tierischen Ektoparasiten und von Hygieneschädlingen vorzugsweise aus der Ordnung Diptera und den Falmilien Culicidae, Simuliidae, Tipulidae, Muscidae und Calliphoridae eingesetzt. Als besonders wirksam erweisen sich dabei die Verbindungen der Formel I gegen Larven der zur Familie Calliphoridae gehörenden Blowfly (Lucilia sericata und Lucilia cuprina) sowie gegen Fliegen- und Mückenelarven.

.Weiterhin sind die Verbindungen der Formel I wirksam gegen Vertreter der Ordnungen Siphonaptera (z.B.

blutsaugende Flöhe).

Neben ihrer Wirkung gegen Mücken und Fliegen, wie z.B. Aedes aegypti und Musca domestica, können Verbindungen der Formel I auch zur Bekämpfung von pflanzenschädigenden Frassinsekten in Zier- und Nutzpflanzungen, insbesondere in Reiskulturen (z.B. gegen Nilaparvata lungens und Nephotettix cincliceps erfolgreich eingesetzt werden.

In ihrer Aktivität zeigen die erfindungsgemässen Verbindungen eine Wirkungsbreite, die über das Karvenstadium hinausgehend auch die übrigen Entwicklungsstadien der Parasiten sowie die Ablage fertiler Eier umfast.

Darüber hinaus zeichnen sich die Verbindungen der Formel I in völlig überraschender Weise durch eine biologische Langzeitwirkung, aus, die ein besonders Merkmal dieser Verbindungen darstellt. Diese prolongierende Wirkungsweise kann sich je nach Anwendungsart über einen Zeitraum von mehr als 3 Monaten erstrecken, was gegenüber bekannten Präparaten vielfältige Vorteile bietet.

Beim Stall-hygienischen Einsatz der erfindungsgemässen Wirkstoffe wird dadurch beispielsweise eine äusserst niedrige Anwendungsfrequenz erreicht, sodass in gemässigten Klimazonen mit 3-monatiger Sommersaison eine einmalige Anwendung genügt, um eine durch die klimatischen Bedingungen normalwerweise begünstigte Entwicklung der schädlichen Dipterenlarven in den Stallungen nachhaltig zu hemmen.

Bei der Behandlung von Weidetieren mit den erfindungsgemässen Verbindungen, z.B. mittels Viehbäder, Aufguss-Methoden oder Sprühgänge, wird durch die überraschende Adhäsionswirkung der Aktivsubstanzen ein lang anhaltender toxischer Effekt gegen Ektoparasiten, wie beispielsweise schädliche Dipteren, auf der Haut und dem Fell der Tiere erzielt. Ein frühzeitiges Aus- oder Abwaschen der auf die Oberfläche der Nutztiere applizierten Wirkstoffe durch ablaufendes Regenwasser kann somit verhindert wrden.

Der besondere Vorteil der verlängerten Wirkung der Verbindungen der Formel I wirkt sich vor allem bei der oralen Verabreichung an Nutztiere aus. Bei diesem Anwendungsverfahren entfalten die Wirkstoffe vor allem in den ausgeschiedenen Fäkalien eine nachhaltige und langandauernde insektizide Aktivität. Dadurch kann der Befall mit schädlichen Insekten, insbesondere Dipteren, bereits vor dem Auftreten der Schädlinge in der Umgebung der Tiere, wie Stallungen, Gehegen und Weide, verhindert werden, weil die aus den abgelegten Eiern schlüpfenden Dipterenlarven sofort abgetötet werden. Bei dieser speziellen Anwendung ist besonders bedeutsam, dass sich die Verbindungen der Formel I aufgrund ihrer strukturellen Eigenschaften gegenübr Warmblütern physiologisch indifferent verhalten. Diese Methode der gezielten Bekämpfung der Proliferation der Insekten ist bedeutend effizienter und gleichzeitig ökonomischer und umweltfreundlicher als die üblichen grossflächigen Behandlungen von Stallungen und Gehegen.

Zurn Bekämpfung der Schädlinge werden die erfindungsgemässen Verbindungen der Formel I sowohl allein als auch in Form von Mitteln eingesetzt, welche noch geeignete Trägerstoffe und Zuschlagstoffe oder Gemische solcher Stoffe enthalten. Geeignete Träger- und Formulierungshilfasstoffe können fest oder flüssig sein und entsprechen den in der Formulierungstechnik üblichen Stoffen, wie z.B. natürlichen oder regenerierten Stoffen, Lösungs-, Dispergier-, Netz-, Haft-, Verdickungs- oder Bindemitteln.

Zur Applikation werden die Verbindungen der Formel I zu Stäubemitteln, Emulsionskonzentraten, Granulaten, Dispersionen, Sprays, zu Ködern, zu Premixturen und zu Lösungen oder Aufschlämmungen in üblichen Formulierungen, die in der Applikationstechnik zum Allgemeinweissen gehören, verarbeitet.

Die orale Applikation der erfindungsgemässen Wirkstoffe der Formel I an Nutztiere erfolgt zweckmässigerweise in Aufwandmengen von 0,1 bis 1000, vorzugsweise 2 bis 100 mg pro Kilogram Körpergewicht. Werden diese Substanzen hingegen topisch auf die Nutztiere appliziert, so liegen die günstigen Wirkstoffkonzentrationen bei 1 bis 5000, bevorzugt 100 bis 1000 ppm. Setzt man die erfindungsgemässen Substanzen im Hygiene-Bereich oder im Pflanzenschutz ein, so dass sie auf gewisse Flächen appliziert werden müssen, so verwendet man vorteilhafterweise Konzentrationen von 1 bis 1000, insbesondere 10 bis 500 ppm.

Die Herstellung der erfindungsgemässen Mittel erfolgt in an sich bekannter Weise durch inniges Vermischen und/oder Vermahlen von Wirkstoffen der Formel mit geeigneten Trägerstoffen, gegebenen falls unter Zusatz von gegenüber den Wirkstoffen inerten Dispergierund Lösungsmitteln. Die Wirkstoffe können in den folgenden Aufarbeitungsformen vorliegen und angewendet werden.

Feste Aufarbeitungsformen: Stäubemittel, Streumittel, Granulate, Premix, Köder, (Umhüllungsgranulate, Imprägnierungsgranulate und Homogengranulate).

Flüssige Aufarbeitungsformen:

a) in Wasser dispergierbare Wirkstoffkonzentrate: Spritzpulver (wettable powder), Pasten, Emulsionen;

b) Lösungen: Aufguss, Sprühmittel.

Der Gehalt an Wirkstoff in den vorstehend beschriebenen Applikationsformen liegt zwischen 0,1 und 95,0 Gew.%, vorzugsweise zwischen 1 und 80 Gew.%.

Die erfindungsgemässen Verbindungen der Formel I sind als Wirkstoffe von Mitteln durch die verschiedenen Zubereitungsformen in vielfältiger Weise zur Bekämpfung von Parasiten auf oder in der Umgebung von Tieren, z.B. in Tierstallungen, geeignet. So können sie beispeilsweise in Viehbädern (cattle dips), Sprühgängen (spray races), Aufgusslösungen (pour-on) oder Handsprühmitteln angewendet werden. Ferner sind sie mit gutem Erfolg für die Behandlung von tierischen Fäkalien mittels der "Feed-through"-Methode und die stall-hygienische Mistbehandlung einsetztbar.

Herstellungsbeispiele:

Beispiel H1: Herstellung von 2-Cyclopropylamino-4,6-bis-isobutyoxy-carbonylamino-s-triazin

Chlorameisensäureisobutylester (16,4 g, 0,12 mol) wird zu einem Gemisch aus 8,3 g 2,4-Diamino-6-cyclopropylamino-s-triazin (0,05 mol), 0,05 g 4-Dimethylaminopyridin und 125 ml Pyridin getropft. Es wird 1 Stunde bei Raumtemperatur und anschliessend 1 Stunde bei 55-60°C gerührt.

Nach dem Abfiltrieren des Niederschlages wird im Wasserstrahlvakuum eingedampft und der Rückstand in 150 ml Chloroform aufgenommen und mit 100 ml Wasser ausgeschüttelt. Nach dem Trocknen über Natriumsulfat wird das Lösungsmittel abdestilliert und das Rohprodukt durch Säulenchromatgraphie (Silica, Methylenchlorid) gereinigt.

Es werden 6,2 g des Produktes erhatlen. Smp. 146-147°C.

Beispiel H2: Herstellung von 2-Cyclopropylamino-4-amino-6-allyloxycarbonylamino-s-triazin

Chlorameisensäureallylester (6,6 g, 0,055 mol) in 50 ml Dioxan gelöst, wird langsam innerhalb 4 Stunden bei Raumtemperatur zu einem Gemich von 6,6 g 2,4-Diamino-6-cyclopropylamino-3-triazin (0,05 mol), 5,1 g Triäthylamin (0,05 mol) und 300 ml Dioxan zugetropft.

Es wird anschliessend über Nacht bei Raumtemperatur gerührt. Nach dem Abfiltrieren des Niederschlages wird im Wasserstrahlvakuum eingedampft und der Rückstand in 400 ml Tetrahydrofuran aufgenommen und mit 200 ml Wasser und 150 ml gesättigter Kochsalzlösung ausgeschüttelt.

Nach dem Trocknen über Magnesiumsulfat wird das Lösungsmittel abgedampft.

Der Rückstand wird in 50 ml Aceton aufgenommen. Unter gutem Rührem wird das Produkt mit Essigester ausgefällt, filtriert und getrocknet.

Ausbeute: 8,8 g. Smp.: 168-171°C.

Beispiel H3: Herstellung von 2-Cyclopropylamino-4-amino-6-allyloxycarbonylamino-s-triazin Hydrochlorid

250 mg (1 mmol) der freien Base werden bei Raumtemperature in 0,55 ml (1,1 mmol) wässriger, 2-normaler Salzsäure aufgelöst und während 10 Minuten kräftig gerührt. Anschliessend wird am Hochvakuum zur Trockne eingedampft.

Das gebildete amorphe Hydrochloridsalz schmilzt oberhalb von 68°C.

Analog zu den beschriebenen Arbeitsweisen können unter anderem auch folgende Verbindungen der Formel I hergestellt werden.

Tabelle 1: Verbindungen der Formel I

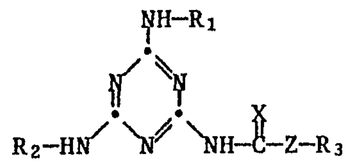

(I)

[Z = Zersetzung]

| Verb. Nr. | $R_1$ | $R_2$ | 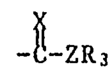 | Salz | Physikalische Konstante [°C] |
|---|---|---|---|---|---|
| 1.1 | Cyclopropyl | $CO-OCH_3$ | $CO-OCH_3$ | --- | Smp. 75-80 |
| 1.2 | Cyclopropyl | H | $CO-OCH_3$ | --- | Smp. 152-155 |
| 1.3 | Cyclopropyl | H | $CO-OCH_3$ | HCl | Smp. ab 98 Z |
| 1.4 | Cyclopropyl | $CO-OCH_2CH=CH_2$ | $CO-OCH_2CH=CH_2$ | --- | Smp. 68-70 |
| 1.5 | Cyclopropyl | $CO-OCH_2CH=CH_2$ | $CO-OCH_2CH=CH_2$ | HCl | Smp. 65-68 |
| 1.6 | Cyclopropyl | $CO-OC_2H_5$ | $CO-OC_2H_5$ | --- | Smp. 93-97 |
| 1.7 | Cyclopropyl | $CO-OC_2H_5$ | $CO-OC_2H_5$ | HCl | Smp. 55-60 |
| 1.8 | Cyclopropyl | H | $CO-OC_2H_5$ | --- | Smp. 208-210 |
| 1.9 | Cyclopropyl | H | $CO-OC_2H_5$ | HCl | Smp. 108-113 |

Tabelle 1: Verbindungen der Formel I (Fortsetzung):

| Verb. Nr. | $R_1$ | $R_2$ | $-\overset{X}{\overset{\|}{C}}-ZR_3$ | Salz | Physikalische Konstante [°C] |
|---|---|---|---|---|---|
| 1.10 | Cyclopropyl | H | $CO-OCH_2CH=CH_2$ | --- | Smp. 178-182 |
| 1.11 | Cyclopropyl | H | $CO-OCH_2CH=CH_2$ | HCl | Smp. 63-68 |
| 1.12 | Cyclopropyl | $CO-OC_2H_5$ | $CO-OC_2H_5$ | HBr | Smp. ab 90 Z |
| 1.13 | Cyclopropyl | H | $COC_4H_9$-n | --- | Smp. 184-185 |
| 1.14 | Cyclopropyl | $CO-OCH_2CH(CH_3)_2$ | $CO-OCH_2CH(CH_3)_2$ | --- | Smp. 146-147 |
| 1.15 | Cyclopropyl | $CO-OC_4H_9$-n | $CO-OC_4H_9$-n | --- | Smp. 127-129 |
| 1.16 | Cyclopropyl | $CO-OC(CCl_3)(CH_3)_2$ | $CO-OC(CCl_3)(CH_3)_2$ | --- | Smp. ab 155 Z |
| 1.17 | Cyclopropyl | H | $CO-OC(CH_3)_2CCl_3$ | --- | |
| 1.18 | Cyclopropyl | H | $CO-OC(CH_3)_2CCl_3$ | HCl | |
| 1.19 | Cyclopropyl | H | $CO-OC_4H_9$-i | --- | |
| 1.20 | Cyclopropyl | H | $CO-OC_4H_9$-i | HCl | |
| 1.21 | i-Propyl | H | $CO-OCH_3$ | --- | |

<u>Tabelle 1</u>: Verbindungen der Formel I (Fortsetzung):

| Verb. Nr. | $R_1$ | $R_2$ | $-\overset{X}{\overset{\|}{C}}-ZR_3$ | Salz | Physikalische Konstante [°C] |
|---|---|---|---|---|---|
| 1.22 | i-Propyl | H | $CO-OCH_3$ | HCl | |
| 1.23 | i-Propyl | Cyclopropyl | $CO-OC_2H_5$ | --- | |
| 1.24 | i-Propyl | Cyclopropyl | $CO-OC_2H_5$ | HCl | |

Beispiel B1: Wirkung gegen Lucilia sericata

Frisch abgelegte Eier der Schmeissfliege L. sericata werden in kleinen Portionen (30-50 Eier) in Reagenzgläser gegeben, in denen zuvor 4 ml Nährmedium mit 1 ml Testlösung in der für die Endkonzentration notwendigen Zwischenverdünnung vermischt worden sind. Nach der Beimpfung des Kulturmediums werden die Testgläser mit einem Wattestopfen verschlossen und im Brutschrank bei 30°C über 4 Tage bebrütet. In dem zum Vergleich unbehandelten Medium entwickeln sich bis zu diesem Zeitpunkt ca. 1 cm lange Larven (Stadium 3). Ist eine Substanz aktiv, so sind die Larven zu diesem Zeitpunkt entweder tot oder moribund und deutlich zurückgeblieben. Der Versuch wird simultan mit Konzentrationen von 100 - 0.01 ppm durchgeführt. Die Wirksamkeit wird gemessen an der niedrigsten noch voll wirksamen Konzentration (LC 100).

Der Test erfasst sowohl durch Kontakt als auch als Frassgift wirksame Substanzen. Auch Repellenz wird berücksichtigt, da die Larven aus dem Medium auswandern und verhungern.

Eine Reihe von Verbindungen der Formel I aus Tabelle 1 sind bei Konzentrationen von 0.1 bis 5.0 ppm voll wirksam. So zeigten z.B. die Verbindungen Nr. 1.3, 1.5, 1.10, 1.11 und 1.13 volle Wirkung.

Beispiel B2: Wirkung gegen Lucilia cuprina

Frisch abgelegte Eier der Schmeissflieege L. curpina werden in kleinen Portionen (30-50 Eier) in Reagenzgläser gegeben, in denen zuvor 4 ml Nährmedium mit 1 ml Testlösung in der für die Endkonzentration notwendigen Zwischenverdünnung vermischt worden sind. Nach der Beimpfung des Kulturmediums werden die Testgläser mit einem Wattestopfen verschlossen und im Brutschrank bei 30°C über 4 Tage bebrütet.

In dem zum Vergleich unbehandelten Medium entwickeln sich bis zu diesem Zeitpunkt ca. 1 cm lange Larven (Stadium 3). Ist eine Substanz aktiv, so sind die Larven zu diesem Zeitpunkt entweder tot oder moribund und deutlich zurückgeblieben. Der Versuch wird simultan mit Konzentrationen von 100 - 0.01 ppm durchgeführt. Die Wirksamkeit wird gemessen an der niedrigsten noch voll wirksamen Konzentration (LC 100).

Der Test erfasst sowohl durch Kontakt als auch als Frassgift wirksame Substanzen. Auch Repellenz wird berücksichtigt, da die Larven aus dem Medium auswandern und verhungern.

Eine Reihe von Verbindungen aus Tabelle 1 sind bei Konzentrationen von 0,1 bis 5,0 ppm voll wirksam, wie z.B. Nr. 1.3, 1.8, 1.9, 1.10 und 1.11.

Beispiel B3: Wirkung gegen Aedes aegypti

Auf der Oberfläche von 150 ml Wasser, dass sich in einem Behälter befindet, wird so viel einer 0,1%igen acetonischen Lösung des Wirkstoffes pipettiert, dass Konzentrationen von je 10, 5 und 1 ppm erhalten werden. Nach Verdunsten des Acetons wird der Behälter mit 50-100 3-tägigen Aedes Larven beschickt. Nach 1 und 8 Tagen wird die Mortalitätsrate ermittelt.

Einige Verbindungen gemäss Tabelle 1 bewirken bei Aufwandmengen zwischen 5 und 10 ppm eine 100 % Abtötung der Larven.

Beispiel B4: Wirkung gegen Nilaparvate lugens (Nymphen) und Nephotettix cincliceps

Der Test wird an wachsenden Reis-Pflanzen durchgeführt. Dazu werden jeweils 10 Pflanzen (Dicke des Stengels 8 mm; ca, 20 cm) in Töpfe (Durchmesser von 5,5 cm) eingepflanzt.

Die Pflanzen werden auf einem Drehteller mit jeweils 50 ml einer wässrigen Lösung enthaltend 400 oder 800 ppm des formulierten Wirkstoffes besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein Glaszylinder gestülpt und dieser mit einem Gaze-Deckel abgedeckt. Die Nymphen werden bis zum Erreichen des Adultenstadiums über 6 Tage an der behandelten Pflanze gehalten. Die Bestimmung der Mortalität erfolgt 6 Tage nach der Behandlung.

Verbindungen aus Tabelle 1 zeigen gegen Nymphen von Nilaparvata lugens und von Nephotettix cincliceps gute Wirksamkeit.

Wirkstoff-Formlierungen

Die Wirkstoffe der Formel I können beipielsweise wie folgt formuliert werden:

Granulat

5 Teile Wirkstoff aus Tabelle 1
0,25 Teile epoxydiertes Pflanzenöl,
0,25 Teile Cetylpolyglykoläther,
3,50 Teile Polyäthylenglykol
91 Teile Kaolin (Korngrösse 0,3-0,8 mm).

Die Aktivsubstanz wird mit dem epoxydierten Pflanzenöl in 6 Teilen Aceton gelöst, hierauf wird Polyäthylenglykol und Cetylpolyglykoläther zugesetzt. Die so erhaltene Lösung wird auf Koalin aufgesprüht und anschliessend das Aceton im Vakuum verdampft. Solche Granulate können dem Viehfutter zugesetzt werden.

Stäubemittel

5 Gew.-Teile feingemahlener Wirkstoff aus Tabelle 1 werden mit
2 Gew.-Teilen einer gefällten Kieselsäure und

93 Gew.-Teilen Talk intensiv gemischt.

Der Wirkstoff wird mit den Trägerstoffen homogen gemischt und vermahlen. Das Stäubemittel kann nicht äusserlich, sondern auch als Zusatz zum Viefutter angewendet werden.

Spritzpulver

5 bis 30 Gew.-Teile Wirkstoffe aus Tabelle 1 werden in einer Mischapparatur mit
5 Gew.-Teilen eines aufsaugenden Trägermaterials (Kieselsäure K 320 oder Wessalon S) und
55 bis 80 Gew.-Teilen eines Trägermaterials [Bolus alba oder Kaolin (B 24)] und einem Dispergiermittelgemisch, bestehend aus
5 Gew.-Teilen Na-Laurylsulfonats und
5 Gew.-Teilen eines Alkyl-aryl-polyglykoläther, intensiv vermischt.
Diese Mischung wird in einer Stift- oder Luftstrahlmühle bis auf 15-15 µm gemahlen. Das so erhaltene Spritzpulver gibt in Wasser eine gute Suspension.

Emulsionskonzentrat

20 Gew.-Teile Wirkstoff aus Tablelle 1 werden in
70 Gew.-Teilen Xylol gelöst und mit
10 Gew.-Teilen eines Emulgiermittels, bestehend aus einem Gemisch eines Alkylphenylpolyglykoläthers und dem Calciumsalz der Dodecylbenzolsulfonsäure, versetzt.
Das Emulsionskonzentrat kann in beliebigem Verhältnis mit Wasser versetzt werden und bildet dabei eine milchige Emulsion, die den Viehtränken zugesetzt werden kann.

Aufguss-Lösung (pour-on)

| | |
|---|---|
| Wirkstoff aus Tabelle 1 | 30,00 g |
| Natrium Dioctylsulfosuccinat | 3,00 g |
| Benzylalkohol | 35,46 g |
| Aethylenglykol-monomethyläther | 35,46 g |
| | 103,92 g = 100 ml |

Die Wirksubstanz wird in dem grössten Teil des Gemisches der beiden Lösungsmittel unter kräftigem Rühren gelöst. Anschliessend wird das Natrium-dioctylsulfosuccinat, eventuell unter Erwärmen, gelöst und schliesslich mit dem restlichen Lösungsmittelgemisch aufgefüllt.

**Patentansprüche**

1. Verbindungen der Formel I

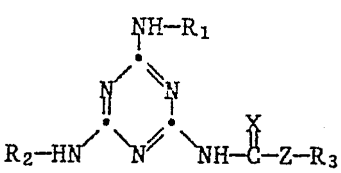

(I)

worin
$R_1$ $C_1$-$C_6$-Alkyl oder $C_3$-$C_6$- Cycloalkyl bedeutet;
$R_2$ für Wasserstoff, $C_1$-$C_6$-Alkyl, $C_3$-$C_6$-Cycloalkyl oder die Gruppe -C(X)-Z-$R_3$ steht;
$R_3$ für $C_1$-$C_6$-Alkyl, $C_1$-$C_6$-Haloalkyl, $C_2$-$C_4$-Alkenyl oder $C_2$-$C_4$-Haloalkenyl steht;
X für Sauerstoff oder Schwefel steht; und
Z für Sauerstoff oder Schwefel steht;
unter Einschluss deren Säureadditionssalze.

2. Verbindungen der Formel I nach Anspruch 1, dadurch gekennzeichnet, dass
$R_1$ $C_3$-Alkyl oder Cylcopropyl bedeutet;
$R_2$ für Wasserstoff, $C_1$-$C_3$-Alkyl, Cyclopropyl oder C(O)O$R_3$ steht;
$R_3$ $C_1$-$C_4$-Alkyl, $C_1$-$C_4$-Chloroalkyl, $C_2$-$C_4$-Alkenyl oder $C_2$-$C_4$-Haloalkenyl bedeutet; und
X und Z für Sauerstoff stehen.

3. Verbindungen der Formel I nach Anspruch 2, dadurch gekennzeichnet, dass
$R_1$ Isopropyl oder Cyclopropyl bedeutet;
$R_2$ für Wasserstoff oder $C_1$-$C_3$-Alkyl steht; $R_3$ Methyl, Ethyl, n-Propyl, n-Butyl oder $C_2$-$C_4$-Alkenyl bedeutet; und
X und Z für Sauerstoff stehen.

4. Eine Verbindung der Formel I nach Anspruch 3, ausgewählt aus der Reihe
2-Cyclopropylamino-4-amino-6-allyloxycarbonyl-amino-s-triazin und seine Säureadditionssalze,
2-Isopropylamino-4-amino-6-allyloxycarbonyl-amino-s-triazin und seine Säureadditionssalze,
2-Cyclopropylamino-4-amino-6-methoxycarbonylamino-s-triazin und seine Säureadditionssalze,
2-Cyclopropylamino-4-amino-6-ethoxycarbonylamino-s-triazin und seine Säureadditionssalze,
2-Cyclopropylamino-4-amino-6-n-butoxycarbonylamino-s-triazin und seine Säureadditionssalze.

Verfahren zur Herstellung einer Verbindung der Formel I

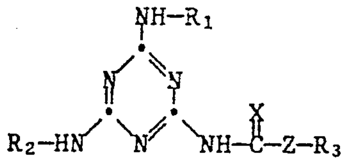

(I)

worin
$R_1$ $C_1$-$C_6$-Alkyl oder $C_3$-$C_6$-Cycloalkyl bedeutet;
$R_2$ für Wasserstoff, $C_1$-$C_6$-Cycloalkyl oder die Gruppe -C(X)-Z-$R_3$ steht;
$R_3$ für $C_1$-$C_6$-Alkyl, $C_1$-$C_6$-Haloalkyl, $C_2$-$C_4$-Alkenyl oder $C_2$-$C_4$-Haloalkenyl steht;
X für Sauerstoff oder Schwefel steht; und
Z für Sauerstoff oder Schwefel steht;
unter Einschluss deren Säureadditionssalze, dadurch gekennzeichnet, dass man eine Verbindung der Formel II

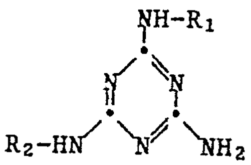

(II),

vorzugsweise in Gegenwart einer Base, mit einer ausreichenden Menge einer reaktionsfähigen Säurederivates der Säure III

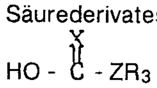 (III)

bei Temperaturen von 0° bis 50°C, vorzugsweise 15° bis 30°C, versetzt und das resultierende Reaktionsgemisch ca. 5 bis 12 Stunden bei 10° bis 100°C hält, wobei in den Formeln II und III die Substituenten $R_1$, X, Z und $R_3$ die unter Formel I angegebenen Bedeutungen haben und $R_2$ für Wasserstoff, $C_1$-$C_6$-Alkyl oder $C_3$-$C_6$-Cycloalkyl steht.

6. Verfahren nach Anspruch 5, dadurch gekennzeichnet, dass man als reaktionsfähiges Derivat der Säure III ihr Anhydrid oder Säurehalogenid einsetzt.

7. Verfahren nach Anspruch 6, dadurch gekennzeichnet, dass man die Reaktion in einem reaktionsinerten Lösungsmittel durchführt.

8. Mittel gegen tier- und/oder pflanzenparasitäre Schädlinge, dadurch gekennzeichnet, dass es neben üblichen Formulierungshilfsstoffen als aktive Komponente mindestens eine Verbindung der Formel I enthält,

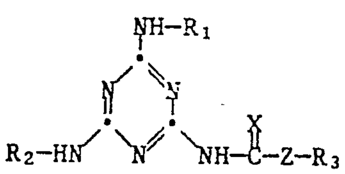

(I)

worin
$R_1$ $C_1$-$C_6$-Alkyl oder $C_3$-$C_6$-Cycloalkyl bedeutet;
$R_2$ für Wasserstoff, $C_1$-$C_6$-Alkyl, $C_3$-$C_6$-Cycloalkyl oder die Gruppe -C(X)-Z-$R_3$ steht;
$R_3$ für $C_1$-$C_6$-Alkyl, $C_1$-$C_6$-Haloalkyl, $C_2$-$C_4$-Alkenyl oder $C_2$-$C_4$-Haloalkenyl steht;
X für Sauerstoff oder Schwefel steht; und
Z für Sauerstoff oder Schwefel steht;

unter Einschluss deren Säureadditionssalze.

9. Mittel nach Anspruch 8, dadurch gekennzeichnet, dass es eine Verbindung der Formel I nach einem der Ansprüche 2 bis 4 enthält.

10. Verfahren zur Bekämpfung von Schadinsekten und/oder Ekto-und/oder Endoparasiten, dadurch gekennzeichnet, dass man eine wirksame Menge einer Verbindung der Formel I

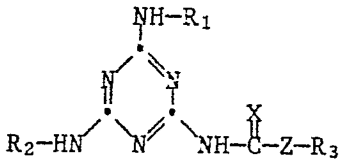

(I)

worin
$R_1$ $C_1$-$C_6$-Alkyl oder $C_3$-$C_6$-Cycloalkyl bedeutet;
$R_2$ für Wasserstoff, $C_1$-$C_6$-Alkyl, $C_3$-$C_6$-Cycloalkyl oder die Gruppe -C(X)-Z-$R_3$ steht;
$R_3$ für $C_1$-$C_6$-Alkyl, $C_1$-$C_6$-Haloalkyl, $C_2$-$C_4$-Alkenyl oder $C_2$-$C_4$-Haloalkenyl steht;
X für Sauerstoff oder Schwefel steht; und
Z für Sauerstoff oder Schwefel steht;
unter Einschluss deren Säureadditionssalze auf den Schädling oder dessen Lebensraum appliziert.

11. Verfahren nach Anspruch 10, dadurch gekennzeichnet, dass zur Behandlung ausgeschiedener Fäkalien die Verbindung der Formel I oral an Nutztieren verabreicht wird.

12. Verfahren nach Anspruch 10, dadurch gekennzeichnet, dass man die Verbindung der Formel I auf die ausgeschiedenen Fäkalien appliziert.

Patentansprüche für den folgenden Vertragsstaat: AT

1. Mittel gegen tier- und/oder pflanzenparasitäre Schädlinge, dadurch gekennzeichnet, dass es neben üblichen Formulierungshilfsstoffen als aktive Komponente mindestens eine der Verbindungen der Formel I

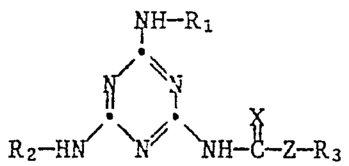

(I)

worin
$R_1$ $C_1$-$C_6$-Alkyl oder $C_3$-$C_6$- Cycloalkyl bedeutet;
$R_2$ für Wasserstoff, $C_1$-$C_6$-Alkyl, $C_3$-$C_6$-Cycloalkyl oder die Gruppe -C(X)-Z-$R_3$ steht;
$R_3$ für $C_1$-$C_6$-Alkyl, $C_1$-$C_6$-Haloalkyl, $C_2$-$C_4$-Alkenyl oder $C_2$-$C_4$-Haloalkenyl steht;
X für Sauerstoff oder Schwefel steht; und
Z für Sauerstoff oder Schwefel steht;
unter Einschluss deren Säureadditionssalze, enthält.

2. Mittel nach Anspruch 1, enthaltend mindestens eine der Verbindungen der Formel I worin
$R_1$ $C_3$-Alkyl oder Cylcopropyl bedeutet;
$R_2$ für Wasserstoff, $C_1$-$C_3$-Alkyl, Cyclopropyl oder C(O)O$R_3$ steht;
$R_3$ $C_1$-$C_4$-Alkyl, $C_1$-$C_4$-Chloroalkyl, $C_2$-$C_4$-Alkenyl oder $C_2$-$C_4$-Haloalkenyl bedeutet; und
X und Z für Sauerstoff stehen.

3. Mittel nach Anspruch 2, enthaltend mindestens eine der Verbindungen der Formel I worin
$R_1$ Isopropyl oder Cyclopropyl bedeutet;
$R_2$ für Wasserstoff oder $C_1$-$C_3$-Alkyl steht;
$R_3$ Methyl, Ethyl, n-Propyl, n-Butyl oder $C_2$-$C_4$-Alkenyl bedeutet; und
X und Z für Sauerstoff stehen.

4. Mittel nach Anspruch 3, enthaltend mindestens eine der Verbindungen der Formel I, ausgewählt aus der Reihe:
2-Cyclopropylamino-4-amino-6-allyloxycarbonyl-amino-s-triazin und seine Säureadditionssalze,
2-Isopropylamino-4-amino-6-allyloxycarbonyl-amino-s-triazin und seine Säureadditionssalze,
2-Cyclopropylamino-4-amino-6-methoxycarbonylamino-s-triazin und seine Säureadditionssalze,
2-Cyclopropylamino-4-amino-6-ethoxycarbonylamino-s-triazin und seine Säureadditionssalze,
2-Cyclopropylamino-4-amino-6-n-butoxycarbonylamino-s-triazin und seine Säureadditionssalze.

5. Verfahren zur Herstellung einer Verbindung der Formel I

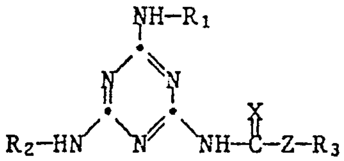

(I)

worin
$R_1$ $C_1$-$C_6$-Alkyl oder $C_3$-$C_6$-Cycloalkyl bedeutet;
$R_2$ für Wasserstoff, $C_1$-$C_6$-Cycloalkyl oder die Gruppe -C(X)-Z-$R_3$ steht;
$R_3$ für $C_1$-$C_6$-Alkyl, $C_1$-$C_6$-Haloalkyl, $C_2$-$C_4$-Alkenyl oder $C_2$-$C_4$-Haloalkenyl steht;
X für Sauerstoff oder Schwefel steht; und
Z für Sauerstoff oder Schwefel steht;
unter Einschluss deren Säureadditionssalze, dadurch gekennzeichnet, dass man eine Verbindung der Formel II

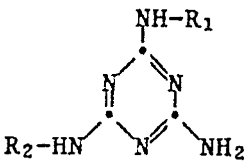

(II),

vorzugsweise in Gegenwart einer Base, mit einer ausreichenden Menge eines reaktionsfähigen Säurederivates der Säure III

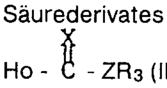

(III)

bei Temperaturen von 0° bis 50°C, vorzugsweise 15° bis 30°C, versetzt und das resultierende Reaktionsgemisch ca. 5 bis 12 Stunden bei 10° bis 100°C hält, wobei in den Formeln II und III die Substituenten $R_1$, X, Z und $R_3$ die unter Formel I angegebenen Bedeutungen haben und $R_2$ für Wasserstoff, $C_1$-$C_6$-Alkyl oder $C_3$-$C_6$-Cycloalkyl steht.

6. Verfahren nach Anspruch 5, dadurch gekennzeichnet, dass man einen der Wirkstoffe aus den Ansprüchen 1 bis 4 herstellt.

7. Verfahren nach Anspruch 5, dadurch gekennzeichnet, dass man als reaktionsfähiges Derivat der Säure III ihr Anhydrid oder Säurehalogenid einsetzt.

8. Verfahren nach Anspruch 6, dadurch gekennzeichnet, dass man die Reaktion in einem reaktionsinerten Lösungsmittel durchführt.

9. Verfahren zur Bekämpfung von Schadinsekten und/oder Ekto-und/oder Endoparasiten, dadurch gekennzeichnet, dass man eine wirksame Menge einer Verbindung der Formel I

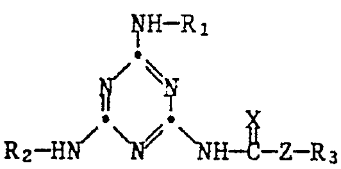

(I)

worin
$R_1$ $C_1$-$C_6$-Alkyl oder $C_3$-$C_6$-Cycloalkyl bedeutet;
$R_2$ für Wasserstoff, $C_1$-$C_6$-Alkyl, $C_3$-$C_6$-Cycloalkyl oder die Gruppe -C(X)-Z-$R_3$ steht;
$R_3$ für $C_1$-$C_6$-Alkyl, $C_1$-$C_6$-Haloalkyl, $C_2$-$C_4$-Alkenyl oder $C_2$-$C_4$-Haloalkenyl steht;
X für Sauerstoff oder Schwefel steht; und
Z für Sauerstoff oder Schwefel steht;
unter Einschluss deren Säureadditionssalze auf den Schädling oder dessen Lebensraum appliziert.

10. Verfahren nach Anspruch 9, dadurch gekennzeichnet, dass zur Behandlung ausgeschiedener Fäkalien die Verbindung der Formel I oral an Nutztieren verabreicht wird.

11. Verfahren nach Anspruch 9, dadurch gekennzeichnet, dass man die Verbindung der Formel I auf die ausgeschiedenen Fäkalien appliziert.

Patentansprüche für den folgenden Vertragsstaat: ES

1. Mittel gegen tier- und/oder pflanzenparasitäre Schädlinge, dadurch gekennzeichnet, dass es neben üblichen Formulierungshilfsstoffen als aktive Komponente mindestens eine der Verbindungen der Formel I

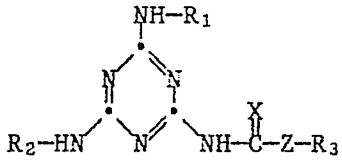

(I)

worin
$R_1$ $C_1$-$C_6$-Alkyl oder $C_3$-$C_6$- Cycloalkyl bedeutet;
$R_2$ für Wasserstoff, $C_1$-$C_6$-Alkyl, $C_3$-$C_6$-Cycloalkyl oder die Gruppe -C(X)-Z-$R_3$ steht;
$R_3$ für $C_1$-$C_6$-Alkyl, $C_1$-$C_6$-Haloalkyl, $C_2$-$C_4$-Alkenyl oder $C_2$-$C_4$-Haloalkenyl steht;
X für Sauerstoff oder Schwefel steht; und
Z für Sauerstoff oder Schwefel steht;
unter Einschluss deren Säureadditionssalze, enthält.

2. Mittel nach Anspruch 1, enthaltend mindestens eine der Verbindungen der Formel I worin
$R_1$ $C_3$-Alkyl oder Cylcopropyl bedeutet;
$R_2$ für Wasserstoff, $C_1$-$C_3$-Alkyl, Cyclopropyl oder C(O)O$R_3$ steht;
$R_3$ $C_1$-$C_4$-Alkyl, $C_1$-$C_4$-Chloroalkyl, $C_2$-$C_4$-Alkenyl oder $C_2$-$C_4$-Haloalkenyl bedeutet; und
X und Z für Sauerstoff stehen.

3. Mittel nach Anspruch 2, enthaltend mindestens eine der Verbindungen der Formel I worin
$R_1$ Isopropyl oder Cyclopropyl bedeutet;
$R_2$ für Wasserstoff oder $C_1$-$C_3$-Alkyl steht;
$R_3$ Methyl, Ethyl, n-Propyl, n-Butyl oder $C_2$-$C_4$-Alkenyl bedeutet; und
X und Z für Sauerstoff stehen.

4. Mittel nach Anspruch 3, enthaltend mindestens eine der Verbindungen der Formel I, ausgewählt aus der Reihe:
2-Cyclopropylamino-4-amino-6-allyloxycarbonyl-amino-s-triazin und seine Säureadditionssalze,
2-Isopropylamino-4-amino-6-allyloxycarbonyl-amino-s-triazin und seine Säureadditionssalze,
2-Cyclopropylamino-4-amino-6-methoxycarbonylamino-s-triazin und seine Säureadditionssalze,
2-Cyclopropylamino-4-amino-6-ethoxycarbonylamino-s-triazin und seine Säureadditionssalze,
2-Cyclopropylamino-4-amino-6-n-butoxycarbonylamino-s-triazin und seine Säureadditionssalze.

5. Verfahren zur Herstellung einer Verbindung der Formel I

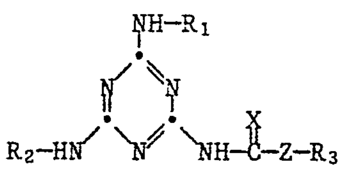

(I)

worin
$R_1$ $C_1$-$C_6$-Alkyl oder $C_3$-$C_6$-Cycloalkyl bedeutet;
$R_2$ für Wasserstoff, $C_1$-$C_6$-Cycloalkyl oder die Gruppe -C(X)-Z-$R_3$ steht;
$R_3$ für $C_1$-$C_6$-Alkyl, $C_1$-$C_6$-Haloalkyl, $C_2$-$C_4$-Alkenyl oder $C_2$-$C_4$-Haloalkenyl steht;
X für Sauerstoff oder Schwefel steht; und
Z für Sauerstoff oder Schwefel steht;
unter Einschluss deren Säureadditionssalze, dadurch gekennzeichnet, dass man eine Verbindung der Formel II

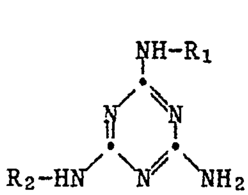

(II),

vorzugsweise in Gegenwart einer Base, mit einer ausreichenden Menge eines reaktionsfähigen Säurederivates der Säure III

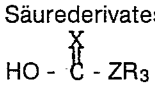 (III)

bei Temperaturen von 0° bis 50°C, vorzugsweise 15° bis 30°C, versetzt und das resultierende Reaktionsgemisch ca. 5 bis 12 Stunden bei 10° bis 100°C hält, wobei in den Formeln II und III die Substituenten $R_1$, X, Z und $R_3$ die unter Formel I angegebenen Bedeutungen haben und $R_2$ für Wasserstoff, $C_1$-$C_6$-Alkyl oder $C_3$-$C_6$-Cycloalkyl steht.

6. Verfahren nach Anspruch 5, dadurch gekennzeichnet, dass man einen der Wirkstoffe aus den Ansprüchen 1 bis 4 herstellt.

7. Verfahren nach Anspruch 5, dadurch gekennzeichnet, dass man als reaktionsfähiges Derivat der Säure III ihr Anhydrid oder Säurehalogenid einsetzt.

8. Verfahren nach Anspruch 6, dadurch gekennzeichnet, dass man die Reaktion in einem reaktionsinerten Lösungsmittel durchführt.